Europäisches Patentamt

European Patent Office

Office européen des brevets

(11) Publication number: **0 501 507 B1**

# EUROPEAN PATENT SPECIFICATION

(45) Date of publication of patent specification: **27.09.95**  (51) Int. Cl.6: **C07C 68/00**, C07C 69/96

(21) Application number: **92103454.2**

(22) Date of filing: **28.02.92**

(54) Process for preparing diester of carbonic acid.

(30) Priority: **01.03.91 JP 57695/91**
**24.01.92 JP 10867/92**

(43) Date of publication of application:
**02.09.92 Bulletin 92/36**

(45) Publication of the grant of the patent:
**27.09.95 Bulletin 95/39**

(84) Designated Contracting States:
**DE ES FR GB IT**

(56) References cited:
**EP-A- 0 425 197**
**EP-A- 0 464 460**

**DATABASE WPIL Week 8543, Derwent Publications Ltd., London, GB; AN 85-267457 & JP-A-60 181 051**

(73) Proprietor: **UBE INDUSTRIES, LTD.**
**12-32, Nishihonmachi 1-chome**
**Ube-shi,**
**Yamaguchi-ken 755 (JP)**

(72) Inventor: **Nishihira, Keigo, c/o Ube Chemical Factory**
**UBE INDUSTRIES, LTD.,**
**1978-10, Oaza Kogushi**
**Ube-shi,**
**Yamaguchi-ken (JP)**
**Inventor: Tanaka, Shuji, c/o Ube Chemical Factory**
**UBE INDUSTRIES, LTD.,**
**1978-10, Oaza Kogushi**
**Ube-shi,**
**Yamaguchi-ken (JP)**
**Inventor: Kodama, Kunioki, c/o Ube Chemical Factory**
**UBE INDUSTRIES, LTD.,**
**1978-10, Oaza Kogushi**
**Ube-shi,**
**Yamaguchi-ken (JP)**
**Inventor: Kaneko, Takayoshi, c/o Ube Chemical Factory**
**UBE INDUSTRIES, LTD.,**
**1978-10, Oaza Kogushi**
**Ube-shi,**
**Yamaguchi-ken (JP)**

(74) Representative: **Hansen, Bernd, Dr.**
**Dipl.-Chem. et al**
**Hoffmann, Eitle & Partner**
**Patent- und Rechtsanwälte,**
**Postfach 81 04 20**
**D-81904 München (DE)**

EP 0 501 507 B1

## Description

This invention relates to a process for preparing a diester of carbonic acid which is useful as a synthesis starting material for an aromatic polycarbonate and various chemical agents. More particularly, it relates to a process for preparing a diester of carbonic acid which comprises reacting carbon monoxide and a nitrite in the presence of a specific catalyst with high yield.

As a method for preparing a diester of carbonic acid, it has been proposed, in Japanese Provisional Patent Publication No. 181051/1985, a method which comprises reacting a nitrite and carbon monoxide in a vapor phase in the presence of a solid catalyst in which a platinum group metal or a compound thereof is carried on a carrier such as an activated charcoal, and in the presence of an oxidising agent in an amount of 10 mole % or more as $O_2$ per carbon monoxide.

However, in this method, in spite of co-existing an oxidizing agent such as oxygen in a ratio as mentioned above to carbon monoxide in order to prevent by-production of a diester of oxalic acid, a significant amount of a diester of oxalic acid is by-produced so that a selectivity of a diester of carbonic acid is low and also the reaction rate is slow. Also, when a mixed gas comprising a nitrite, carbon monoxide, an alcohol and oxygen is supplied to the reaction, there is a fear of explosion and thus, there is a problem in safety.

The present inventors have previously researched about the process for preparing diester of carbonic acid by reacting carbon monoxide and a nitrite in a vapor phase and proposed the method of using a solid catalyst in which a chloride of a platinum group metal and a chloride of a second metal component selected from iron, copper, cobalt, nickel and tin are carried on an activated charcoal (Japanese Patent Application No. 274816/1989 which corresponds to U.S. Serial No. 07/599,134 and European Patent Publication No. 0 425 197) and an improved method thereof in which the above catalyst as well as a minute amount of hydrogen chloride are coexisted in the reaction system (Japanese Patent Application No. 201146/1990 which also corresponds to U.S. Serial No. 07/599,134 and European Patent Publication No. 0 425 197). By these inventions, it can be realized to prepare a diester of carbonic acid with high selectivity and high yield in an industrial scale.

According to the above preparation process, a minute amount of hydrogen chloride is dissipated from the catalyst so that activity of the catalyst is gradually lowered, and thus, a minute amount of hydrogen chloride is added to the reaction system in order to prevent the dissipation. However, according to this method, there are problems that chlorine component is migrated in the product or an apparatus for the reaction is corroded by the chlorine component.

## SUMMARY OF THE INVENTION

An object of the present invention is to solve the above problems caused by the chlorine component and to provide a process for preparing a diester of carbonic acid with high selectivity and high yield under mild reaction conditions by maintaining the activity of the catalyst to a high position for a long period of time.

The present inventors have found that the problems caused by the above chlorine component can be solved by using zeolite in which an acid point is neutralized by an alkali metal or an alkaline earth metal, or exchanged by a hydrogen ion or an ammonium ion, and the alkali metal ion, the alkaline earth metal ion, the hydrogen ion or the ammonium ion is exchanged by a platinum group metal ion and a second component metal ion and carried thereon whereby the present invention has been accomplished.

The present invention is a process for preparing a diester of carbonic acid which comprises bringing carbon monoxide into contact with a nitrite (an ester of nitrous acid) in a vapor phase in the presence of a catalyst comprising

(a) a platinum group metal ion and
(b) at least one ion of a metal selected from the group consisting of copper, iron, tin, nickel, cobalt, cerium, silver and manganese, characterized in that the catalyst is carried on zeolite, and the hydrogen, ammonium, alkali metal or alkaline earth metal counter-ions of the zeolite are exchanged by the metal ions in (a) and (b).

## DESCRIPTION OF THE PREFERRED EMBODIMENTS

In the following, the present invention is to be described in more detail.

The zeolite to be used as a carrier in the present invention may be either synthetic zeolite or natural zeolite and there nay be mentioned, for example, A type zeolite, X type zeolite, Y type zeolite, mordenite and silicalite, preferably A type, X type and Y type, particularly preferably Y type zeolite (having high Si/Al ratio) since it has high thermal resistance.

Zeolite may be powder or granular state, and when it is powder, those having 20 to 100 $\mu$m in the average diameter can be suitably used and when it is granule, those having 4 to 200 mesh in the average diameter can be suitably used.

As the platinum group metal to be carried on zeolite, there may be suitably used palladium, platinum, iridium, ruthenium and rhodium, and palladium is most preferred. As the second component metal, there may be used copper, iron, tin, nickel, cobalt, cerium, silver and manganese, preferably copper, iron and manganese, most preferably copper. These second component metal ions act as a role of like a co-catalyst, and as compared to the catalyst in which the platinum group metal ion alone is used, lifetime of the catalyst can be extended greatly.

For carrying these platinum group and the second component metals on zeolite as ions, a halide such as bromide, chloride and iodide, a nitride, a sulfate, a phosphate, an acetate or an oxalate of these metals can be used. As a method for carrying the metal ion, according to the conventional ion exchange method, an alkali metal ion, an alkaline earth metal ion, hydrogen ion or an ammonium ion of zeolite is exchanged by the above metal ion to carry thereon whereby the catalyst to be used in the present invention can be prepared.

An amount of the metal ion to be carried is, in the case of the platinum group metal ion, 0.1 to 10 % by weight, preferably 1 to 5 % by weight based on zeolite and, in the case of the second component metal ion, 1 to 50 gram atomic equivalents, preferably 1 to 20 gram atomic equivalents based on the platinum group metal.

As a nitrite to be used in the present invention, there may be suitably mentioned a nitrite of a lower aliphatic monohydric alcohol having 1 to 4 carbon atoms such as methyl nitrite, ethyl nitrite, n- (or iso)propyl nitrite and n- (or iso or sec-)butyl nitrite; a nitrite of an alicyclic alcohol such as cyclohexyl nitrite; a nitrite of an aralkyl alcohol such as benzyl nitrite and phenylethyl nitrite; and the like, but particularly preferably a nitrite of a lower aliphatic monohydric alcohol having 1 to 4 carbon atoms, and above all, methyl nitrite and ethyl nitrite are most preferred. Such a nitrite can be easily obtained by oxidizing with oxygen part of $NO_x$ gas obtained by the reaction of sodium nitrite and an acid such as nitric acid, hydrochloric acid and sulfuric acid, or obtained by air oxidation of ammonia, to obtain $NO_x$ gas comprising $NO/NO_2 = 1/1$ (volume ratio) and then contacting an alcohol thereto.

Further, in the process of the present invention, a nitrite generates, after participating the reaction, nitrogen monoxide by decomposition thereof. Thus, it is preferred that the nitrogen monoxide is recovered from a reaction gas led out from a reactor, reacted with oxygen and an alcohol corresponding to a nitrite to be used to convert it to a nitrite again and used by circulation.

As a reaction system of carbon monoxide and a nitrite, it may be carried out in a vapor phase by either a batch system or a continuous system, but the continous system is industrially advantageous. Also, as an existing form of the catalyst in the reaction system, the reaction may be practiced by using a reactor either of a fixed bed, a moving bed or a fluidized bed.

In the present invention, it is desired that carbon monoxide and a nitrite which are starting gases are fed into the above reactor by diluting with an inert gas such as nitrogen gas, etc., and the composition thereof is not particularly limited in view of the reaction. However, from standpoint of safety, a concentration of the above nitrite is desirably 20 % by volume or less, preferably 5 to 20 % by volume. Accompanying with it, a concentration of carbon monoxide is preferably in the range of 5 to 20 % by volume from economical viewpoint. That is, when considering an industrial production process, it is preferred to use gases such as carbon monoxide and a nitrite to be supplied to the reaction in a circulatory system and to purge part of said circulatory gas out of the system. Also, since a conversion of carbon monoxide at one pass is about 20 to 30 %, even if a concentration of carbon monoxide is made more than 20 % by volume, loss will increase, while it is less than 5 % by volume, there are problems that productivity becomes low, and the like. However, if ignoring the economical view point, the concentration of carbon monoxide can be practically heightened up to 80 % by volume. That is, it is also possible to feed a nitrite by diluting carbon monoxide in place of the above inert gas. Accordingly, a ratio of carbon monoxide and a nitrite to be used is desirably in the range of 0.1 to 10 mole, preferably 0.25 to 2 mole of carbon monoxide based on one mole of a nitrite.

In the present invention, a space velocity of a gas containing carbon monoxide and a nitrite to be fed in the above reactor is preferably in the range of 500 to 20000 $h^{-1}$, more preferably in the range of 2000 to 10000 $h^{-1}$.

In the process of the present invention, the contact reaction of carbon monoxide and a nitrite can be carried out under extremely gentle conditions. As the reaction conditions, the reaction can be carried out at a temperature of 0 to 200 °C, preferably 50 to 150 °C under normal pressure. Of course, it can be carried out under pressurized system without any problem and can be carried out at a pressure of 1 to 20 kg/cm²G and a temperature of 50 to 150 °C. Also, when the synthesis of a nitrite is carried out in the above reaction system, the contact reaction of carbon monoxide and a nitrite is particularly preferably carried out in a slightly pressurized system of about 2 to 3 kg/cm²G.

Thus, a reaction gas containing, in addition to a diester of carbonic acid which is the title product, a by-product such as a diester of oxalic acid, unreacted carbon monoxide and a nitrite, nitrogen monoxide, carbon dioxide and an inert gas is led out from the reactor, and after cooling the reaction gas, uncondensed gases such as carbon monoxide, a nitrite, nitrogen monoxide, carbon dioxide and an inert gas are circulated again in the reactor while purging part thereof, and a diester of carbonic acid is separated and purified from a condensate by the conventional method such as distillation.

EXAMPLES

In the following, the process of the present invention will be explained specifically by referring to Examples and Comparative examples, but the present invention is not limited by these.

A space time yield STY (g/ℓ • hr) in each Example or Comparative example is calculated from the following equation:

$$STY = a/(b \times \theta)$$

wherein $\theta$ (hr) is a contact reaction time of carbon monoxide and a nitrite, $\underline{a}$ (g) is an amount of a diester of carbonic acid and $\underline{b}$ (ℓ)is a filled amount of the catalyst to the reactor.

Also, a selectivity X (%) based on carbon monoxide and a selectivity Y (%) based on a nitrite in each Example or Comparative example are calculated by using carbon monoxide or a nitrite to be supplied as standard, and obtained from the following formulae by making amounts (mole) of a diester of carbonic acid, a diester of oxalic acid, carbon dioxide, methyl formate and methylal formed by the above time $\theta$ (hr) as $\underline{c}$, $\underline{d}$, $\underline{e}$, $\underline{f}$, and $\underline{g}$, respectively.

$$X = \{c/(c + 2 \times d + e)\} \times 100$$
$$Y = \{c/(c + d + f + g)\} \times 100$$

Example 1

(Preparation of a catalyst)

In 200 ml of distilled water was dipped 10 g of Y type zeolite substituted by Na ion, and under heating to 70 °C and stirring the mixture, 50 ml of an aqueous solution containing 0.23 g (Pd: 1 mmole) of tetraanminepalladium dichloride was added dropwise over 30 minutes. After maintaining the mixture to 70 °C for 3 hours under stirring, the mixture was cooled to room temperature, filtered and washed with water. After the mixture was dipped again in 200 ml of distilled water, to the mixture was added dropwise 50 ml of an aqueous solution containing 0.68 g (Cu: 5 mmole) of cupric chloride over 30 minutes and the mixture was treated in the same manner as mentioned above. After drying the product at 100 °C for 5 hours, the product was calcinated at 300 °C for 3 hours under air-flow to prepare a catalyst in which palladium ion and copper ion were carried on Y type zeolite.

(Preparation of a diester of carbonic acid)

The above catalyst (5 ml) was filled in a vapor phase reactor (attached with an outer jacket) having an inner diameter of 20 mm, and then the reactor was fixed vertically and a heating medium was circulated in the jacket of the reactor to control a temperature in a catalyst bed to 120 °C. From the upper end of the reactor, a mixed gas comprising a gas containing methyl nitrite synthesized from nitrogen monoxide, oxygen and methanol, and carbon monoxide, i.e. a mixed gas comprising a composition of 15 % by volume of methyl nitrite, 10 % by volume of carbon monoxide, 3 % by volume of nitrogen monoxide, 6 % by volume of methanol and 66 % by volume of nitrogen was supplied with a space velocity (GHSV) of 4000 hr$^{-1}$ to subject the reaction under normal pressure.

Then, the reaction product passed through the reactor was captured by passing through an ice-cold methanol. The captured solution thus obtained was analyzed by gas chromatography to give the results that dimethyl carbonate was formed with STY of 250 g/ℓ • hr, a selectivity based on carbon monoxide of 98 % and a selectivity based on methyl nitrite of 79 %. Also, it was confirmed that even when the reaction was carried out for 50 hours continuously, both of STY and selectivities were not changed at all.

Example 2

(Preparation of a catalyst)

In the same manner as in Example 1 except for using 0.97 g of ferric chloride in place of 0.68 g of cupric chloride, a catalyst in which palladium ion and ferric ion were carried on Y type zeolite was prepared.

(Preparation of a diester of carbonic acid)

By using the above catalyst, reaction was carried out in the same manner as in Example 1. The reaction product passed through the reactor was captured by passing through an ice-cold methanol. The captured solution thus obtained was analyzed by gas chromatography to give the results that dimethyl carbonate was formed with STY of 230 g/ℓ • hr, a selectivity based on carbon monoxide of 97 % and a selectivity based on methyl nitrite of 78 %. Also, it was confirmed that dimethyl oxalate, carbon dioxide and methyl formate were formed as by-products. It was also found that even when the reaction was carried out for 50 hours continuously, catalyst activity was substantially not changed.

Example 3

(Preparation of a catalyst)

In the same manner as in Example 1 except for using 10 g of X type zeolite in place of 10 g of Y type zeolite, a catalyst in which palladium ion and copper ion were carried on X type zeolite was prepared.

(Preparation of a diester of carbonic acid)

By using the above catalyst, reaction was carried out in the same manner as in Example 1. The reaction product passed through the reactor was captured by passing through an ice-cold methanol. The captured solution thus obtained was analyzed by gas chromatography to give the results that dimethyl carbonate was formed with STY of 180 g/ℓ•hr, a selectivity based on carbon monoxide of 95 % and a selectivity based on methyl nitrite of 80 %. Also, it was confirmed that dimethyl oxalate, carbon dioxide and methyl formate were formed as by-products. It was also found that even when the reaction was carried out for 50 hours continuously, catalyst activity was substantially not changed.

Example 4

(Preparation of a catalyst)

In the same manner as in Example 1 except for using 0.49 g (Ce: 2 mmole) of cerium chloride in place of cupric chloride, a catalyst in which palladium ion and cerium ion were carried on Y type zeolite was prepared.

(Preparation of a diester of carbonic acid)

By using the above catalyst, reaction was carried out in the same manner as in Example 1. As the results, it was found that dimethyl carbonate was formed with STY of 400 g/ℓ•hr, a selectivity based on carbon monoxide of 77 % and a selectivity based on methyl nitrite of 79 %. It was also understood that even when the reaction was carried out for 50 hours continuously, catalyst activity was substantially not changed.

Example 5

(Preparation of a catalyst)

In the same manner as in Example 1 except for using 0.34 g of (2 mmole) silver nitrate in place of cupric chloride, a catalyst in which palladium ion and silver ion were carried on Y type zeolite was prepared.

(Preparation of a diester of carbonic acid)

By using the above catalyst, reaction was carried out in the same manner as in Example 1. As the results, it was found that dimethyl carbonate was formed with STY of 380 g/ℓ•hr, a selectivity based on carbon monoxide of 78 % and a selectivity based on methyl nitrite of 78 %. It was also found that even when the reaction was carried out for 50 hours continuously, catalyst activity was substantially not changed.

Example 6

(Preparation of a catalyst)

In the same manner as in Example 1 except for using 0.35 g of (2 mmole) manganese acetate in place of cupric chloride, a catalyst in which palladium ion and manganese ion were carried on Y type zeolite was prepared.

(Preparation of a diester of carbonic acid)

By using the above catalyst, reaction was carried out in the same manner as in Example 1. As the results, it was found that dimethyl carbonate was formed with STY of 410 g/ℓ•hr, a selectivity based on carbon monoxide of 81 % and a selectivity based on methyl nitrite of 81 %. It was also found that even when the reaction was carried out for 50 hours continuously, catalyst activity was substantially not changed.

Comparative example 1

(Preparation of a catalyst)

In 100 ml of 5N hydrochloric acid were dissolved 0.35 g of palladium chloride and 0.34 g of cupric chloride (dihydrate), and after dipping 10 g of granular activated charcoal therein, the mixture was filtered and the filtrate was washed with water and dried at 100 °C to prepare a catalyst (PdCl$_2$-CuCl$_2$/C) carried palladium chloride and cupric chloride on the activated charcoal.

(Preparation of a diester of carbonic acid)

By filling 5 ml of the above catalyst in place of the catalyst of Example 1, reaction was carried out in the same manner as in Example 1. The reaction product passed through the reactor was captured by passing through an ice-cold methanol. The captured solution thus obtained was analyzed by gas chromatography to give the results that dimethyl carbonate was formed with STY of 280 $g/\ell \cdot hr$, a selectivity based on carbon monoxide of 96 % and a selectivity based on methyl nitrite of 93 %. However, continuing the reaction, STY and selectivities were gradually lowered and after 50 hours from initiation of the reaction, STY, a selectivity based on carbon monoxide and a selectivity based on methyl nitrite became 150 $g/\ell \cdot hr$, 85 % and 89 %, respectively.

The vapor phase reaction of carbon monoxide and a nitrite can be carried out under gentle conditions by using a catalyst in which alkali metal ion, alkaline earth metal ion, hydrogen ion or ammonium ion of zeolite is replaced by a platinum group metal ion and a second component metal ion selected from copper, iron, tin, nickel, cobalt, cerium, silver and manganese, whereby a diester of carbonic acid can be obtained with high selectivity and high yield. Also, since the catalyst of the present invention contains no chlorine component, it does not corrode a reaction apparatus and lifetime of the catalyst can be elongated with a great extent.

## Claims

1.  A process for preparing a diester of carbonic acid which comprises bringing carbon monoxide into contact with a nitrite in a vapor phase in the presence of a catalyst comprising
    (a) a platinum group metal ion and
    (b) at least one ion of a metal selected from the group consisting of copper, iron, tin, nickel, cobalt, cerium, silver and manganese, characterized in that the catalyst is carried on zeolite, and the hydrogen, ammonium, alkali metal or alkaline earth metal counter-ions of the zeolite are exchanged by the metal ions in (a) and (b).

2.  The process according to Claim 1, wherein said zeolite is selected from the group consisting of A type zeolite, X type zeolite, Y type zeolite, mordenite and silicalite having an average diameter of 20 to 100 $\mu$m.

3.  The process according to Claim 1, wherein said platinum group metal ion is selected from the group consisting of palladium ion, platinum ion, iridium ion, ruthenium ion and rhodium ion.

4.  The process according to Claim 1, wherein a carried amount of the platinum group metal ion is 0.1 to 10 % by weight based on the weight of zeolite.

5.  The process according to Claim 1, wherein a carried amount of the platinum group metal ion is 1 to 5 % by weight based on the weight of zeolite.

6.  The process according to Claim 1, wherein a carried amount of (b) at least one ion of a metal is 1 to 50 gram atomic equivalent based on an amount of the platinum group metal ion.

7.  The process according to Claim 1, wherein a carried amount of (b) at least one ion of a metal is 1 to 20 gram atomic equivalent based on an amount of the platinum group metal ion.

8.  The process according to Claim 1, wherein said nitrite is selected from the group consisting of methyl nitrite, ethyl nitrite, n-propyl nitrite, isopropyl nitrite, n-butyl nitrite, isobutyl nitrite, sec-butyl nitrite, cyclohexyl nitrite, benzyl nitrite and phenylethyl nitrite.

9.  The process according to Claim 8, wherein said nitrite is methyl nitrite or ethyl nitrite.

10. The process according to Claim 1, wherein a ratio of carbon monoxide and a nitrite is 0.1 to 10 mole of carbon monoxide based on 1 mole of a nitrite.

11. The process according to Claim 1, wherein a space velocity of a gas containing carbon monoxide and a nitrite to be reacted is in the range of 500 to 20000 $h^{-1}$.

12. The process according to Claim 1, wherein said contact is carried out at 0 to 200 °C under normal pressure.

13. The process according to Claim 1, wherein said contact is carried out at 50 to 150 °C at 1 to 20 $kg/cm^2$G.

14. The process according to Claim 1, wherein said contact is carried out under a pressure of 2 to 3 $kg/cm^2$G.

## Patentansprüche

1.  Verfahren zur Erzeugung eines Diesters von Kohlensäure, umfassend das Kontaktieren von Kohlenmonoxid mit einem Nitrit in einer Dampfphase in der Gegenwart eines Katalysators, umfassend

(a) ein Metallion der Platin-Gruppe und

(b) zumindest ein Ion eines Metalls, ausgewählt aus der Gruppe, bestehend aus Kupfer, Eisen, Zinn, Nickel, Kobalt, Cer, Silber und Mangan,

dadurch **gekennzeichnet,** daß der Katalysator auf Zeolith getragen ist und daß die Wasserstoff-, Ammonium-, Alkalimetall- oder Erdalkalimetall-Gegenionen des Zeoliths durch die Metallionen von (a) und (b) ausgetauscht sind.

2. Verfahren nach Anspruch 1, worin der Zeolith ausgewählt aus der Gruppe, bestehend aus Zeolith vom A-Typ, Zeolith vom X-Typ, Zeolith vom Y-Typ, Mordenit und Silicalit, mit einem durchschnittlichen Durchmesser von 20 bis 100 $\mu$m.

3. Verfahren nach Anspruch 1, worin das Metallion der Platin-Gruppe ausgewählt ist aus der Gruppe, bestehend aus Palladiumion, Platinion, Iridiumion, Rutheniumion und Rhodiumion.

4. Verfahren nach Anspruch 1, worin eine getragene Menge des Metallions der Platin-Gruppe 0,1 bis 10 Gew.-% ist, bezogen auf das Gewicht von Zeolith.

5. Verfahren nach Anspruch 1, worin eine getragene Menge des Metallions der Platin-Gruppe 1 bis 5 Gew.-% ist, bezogen auf das Gewicht von Zeolith.

6. Verfahren nach Anspruch 1, worin eine getragene Menge von (b) zumindest einem Ion aus einem Metall 1 bis 50 Grammatomäquivalente ist, bezogen auf eine Menge des Metallions der Platin-Gruppe.

7. Verfahren nach Anspruch 1, worin eine getragene Menge von (b) zumindest einem Ion aus einem Metall 1 bis 20 Grammatomäquivalente ist, bezogen auf eine Menge des Metallions der Platin-Gruppe.

8. Verfahren nach Anspruch 1, worin das Nitrit ausgewählt ist aus der Gruppe, bestehend aus Methylnitrit, Ethylnitrit, n-Propylnitrit, Isopropylnitrit, n-Butylnitrit, Isobutylnitrit, sek-Butylnitrit, Cyclohexylnitrit, Benzylnitrit und Phenylethylnitrit.

9. Verfahren nach Anspruch 8, worin das Nitrit Methylnitrit oder Ethylnitrit ist.

10. Verfahren nach Anspruch 1, worin ein Verhältnis von Kohlenmonoxid und einem Nitrit 0,1 bis 10 mol Kohlenmonoxid ist, bezogen auf 1 mol eines Nitrits.

11. Verfahren nach Anspruch 1, worin eine Raumgeschwindigkeit eines Gases, umfassend Kohlenmonoxid und ein Nitrit, die reagiert werden sollen, in dem Bereich von 500 bis 20 000 h$^{-1}$ ist.

12. Verfahren nach Anspruch 1, worin der Kontakt bei 0 bis 200°C unter Normaldruck durchgeführt wird.

13. Verfahren nach Anspruch 1, worin der Kontakt bei 50 bis 150°C bei 1 bis 20 kg/cm$^2$G durchgeführt wird.

14. Verfahren nach Anspruch 1, worin der Kontakt unter einem Druck von 2 bis 3 kg/cm$^2$G durchgeführt wird.

**Revendications**

1. Procédé pour la préparation d'un diester de l'acide carbonique qui comprend la mise en contact de monoxyde de carbone avec un nitrure en phase vapeur en présence d'un catalyseur comprenant

(a) un ion métallique appartenant au groupe du platine et

(b) au moins un ion d'un métal choisi dans le groupe constitué de cuivre, de fer, d'étain, de nickel, de cobalt, de cérium, d'argent et de manganèse, procédé caractérisé en ce que le catalyseur est imprégné sur de la zéolithe, et en ce que les contre-ions hydrogène, ammonium, de métal alcalin ou alcalino-terreux de la zéolithe sont échangés pour les ions métalliques énumérés dans (a) et (b).

2. Procédé selon la revendication 1, dans lequel la zéolithe est choisie dans le groupe constitué de zéolithe de type A, de zéolithe de type X, de zéolithe de type Y, de mordénite et de silicalite ayant un diamètre moyen de 20 à 100 $\mu$m.

3. Procédé selon la revendication 1, dans lequel l'ion métallique appartenant au groupe du platine est choisi dans le groupe constitué d'ion palladium, d'ion platine, d'ion iridium, d'ion ruthénium et d'ion rhodium.

4. Procédé selon la revendication 1, dans lequel la quantité d'ion métallique appartenant au groupe de platine imprégnée est de 0,1 à 10% en poids sur base pondérale de la zéolithe.

5. Procédé selon la revendication 1, dans lequel la quantité d'ion métallique appartenant au groupe de platine est de 1 à 5% en poids sur base pondérale de la zéolithe.

6. Procédé selon la revendication 1, dans lequel la quantité (b) d'au moins un ion métallique est de 1 à 50 grammes-équivalents atomiques par rapport à la quantité d'ion métallique appartenant au groupe du platine.

7. Procédé selon la revendication 1, dans lequel la quantité (b) d'au moins un ion métallique imprégnée est de 1 à 20 grammes-équivalents atomiques par rapport à la quantité d' ion métallique appartenant au groupe platine.

8. Procédé selon la revendication 1, dans lequel ledit nitrure est choisi dans le groupe constitué de nitrure de méthyle, de nitrure d'éthyle, de nitrure de n-propyle, de nitrure d' isopropyle, de nitrure de n-butyle, de nitrure d'isobutyle, de nitrure de sec-butyle, de nitrure de cyclohexyle, de nitrure de benzyle et de nitrure de phényléthyle.

9. Procédé selon la revendication 8, dans lequel ledit nitrure est le nitrure de méthyle ou le nitrure d'éthyle.

10. Procédé selon la revendication 1, dans lequel le rapport du monoxyde de carbone au nitrure est de 0,1 à 10 moles de monoxyde de carbone pour 1 mole de nitrure.

11. Procédé selon la revendication 1, dans lequel la vitesse spatiale d'un gaz contenant du monoxyde de carbone et un nitrure destinés à réagir s'échelonne de 500 à 20 000 h$^{-1}$.

12. Procédé selon la revendication 1, dans lequel ledit contact est réalisé entre 0 et 200°C à la pression atmosphérique.

13. Procédé selon la revendication 1, dans lequel ledit contact est réalisé entre 50 et 150°C sous une pression manométrique de 1 à 20 kg/cm$^2$.

14. Procédé selon la revendication 1, dans lequel ledit contact est réalisé sous une pression manométrique de 2 à 3 kg/cm$^2$.